# EUROPEAN PATENT SPECIFICATION

(11) **EP 2 984 488 B1**
(45) Date of publication and mention of the grant of the patent: **02.11.2016**
(21) Application number: 14713435.7
(22) Date of filing: 25.03.2014
(51) Int. Cl.: G01N 33/72

(54) **SAMPLE PREPARATION FOR REDUCED VARIABILITY IN RAMAN SPECTROSCOPY**
PROBENVORBEREITUNG FÜR REDUZIERTE VARIABILITÄT IN DER RAMANSPEKTROSKOPIE
PRÉPARATION D'ÉCHANTILLON POUR UNE VARIABILITÉ RÉDUITE EN SPECTROSCOPIE RAMAN

(30) Priority: 10.04.2013 GB 201306494
(43) Date of publication of application: 17.02.2016
(73) Proprietor: Dublin Institute of Technology, Dublin 6 (IE)
(72) Inventor: BONNER, Franck, Dublin 8 (IE); TRAYNOR, Damien, Swords Co. Dublin (IE); LYNG, Fiona, Dublin 7 (IE)
(74) Representative: Walsh, Marie Goretti
(86) International application number: PCT/EP2014/055932
(87) International publication number: WO 2014/166730

(56) References cited:
- WO-A1-2004/070368
- LI ET AL: "Inactivation of Hemoglobin by Hydrogen Peroxide and Protection by a Reductant Substrate", LIFE SCIENCE JOURNAL, vol. 3, no. 1, 25 March 2006 (2006-03-25), pages 52-58, XP055124369,
- BROOKE D. BEIER ET AL: "Method for automated background subtraction from Raman spectra containing known contaminants", THE ANALYST, vol. 134, no. 6, 10 March 2009 (2009-03-10), page 1198, XP055034902, ISSN: 0003-2654, DOI: 10.1039/b821856k

## Description

### Field of the Invention

The present application relates generally to spectroscopy and more particularly to the analysis of biological samples by spectroscopic techniques.

### Background to the Invention

Cervical cancer kills approximately 300,000 women each year worldwide with 80% of the deaths occurring in developing countries. Epithelial abnormalities that are potentially capable of progression into an invasive neoplasm have traditionally been categorised either as dysplasia or as carcinoma in situ. Dysplastic changes within the epithelium are graded as being of a mild, moderate, or severe degree. Those cervical epithelial abnormalities associated with an increased risk of invasive carcinoma have now been classified into a single diagnostic category of cervical intraepitheilial neoplasia (CIN). Three grades of abnormality are recognised: CIN I which corresponds to mild dysplasia; CIN II which is equivalent to moderate dysplasia; and CIN III which encompasses both severe dysplasia and carcinoma in situ. There are two types of cervical cancer: squamous cell cancer and adenocarcinoma. They are named after the type of cell that becomes cancerous. Squamous cell cancer is the most common type of cervical cancer, which occurs in the ectocervix. Adenocarcinoma is cancer of gland cells that are located in the endocervical canal, which is less common than squamous cell cancer. A screening test is used to detect premalignant and maligant processes in the ectocervix. Significant changes can be treated, thus preventing the development of cervical cancer. The main method for cervical cancer screening is the Papanicolaou test (Pap test).

Vibrational spectroscopic techniques are potential tools for the non-invasive, label free investigation of biological samples at a molecular level. Over the course of the last few years, the range of applications of Infrared and Raman spectroscopy has extended from intact tissue to single cell analysis, elucidating different mechanisms involved in malignancy and cancer progression as well as probing the efficiency of new drugs and the toxicity of nanoparticles at cellular level. Vibrational spectroscopy is a fast developing discipline showing a strong potential in the field of cervical cancer screening. Since the first time vibrational spectroscopy was suggested as a tool for mass screening of cervical cancer in the 1990s by Wong *et al.* (1991), there has been significant progress. Numerous studies have shown the potential of vibrational spectroscopic techniques in the detection of cervical cancer and pre-cancerous lesions (Lyng *et al.* 2007, Diem *et al.* 2008). Moreover, spectroscopic data handling and multivariate analysis procedures have significantly been developed over the last two decades.

Li et al. (2006) disclose the inactivation of hemoglobin by hydrogen peroxide, and WO2004/070368 discloses that the signal for the analysis of whole blood by Raman is dependent on the amount of hemoglobin in the sample.

The challenge for vibrational spectroscopy remains for it to be recognized as a medical screening tool that could potentially be used to complement cytology screening techniques. The very high potential to replace the current cytology screening technologies has been proven many times and the high degree of objectivity of the assessment based on molecular composition make them really sensitive and specific tools. Moreover, the identification of specific markers could lead to the capability of detecting early abnormal changes that are not morphologically apparent and thus undetectable using standard methods. Ideally the process employed should be suitable for use for mass screening and accordingly a high degree of automation is desirable.

Extensive studies have been conducted on the study of single cells using Raman spectroscopy. The weak scattering efficiency of water and the high spatial resolution of the visible or near -IR wavelengths employed for Raman microspectroscopy place this technique as the favored tool for the study of living cells, whereby live cells in a physiological solution can be mapped with an immersion lens giving sub cellular resolution of the order of 1 µm. Equipped with high magnification objectives such as x60 or x100, the lateral resolution offered by this technique make it a perfectly suitable tool for single cells analysis.

Nevertheless the quality of the information which may be gleaned requires some improvement. The application of vibrational spectroscopy to biological samples is a complex task, mainly due to the numerous parameters that can possibly affect the interpretation of results. Each patient is unique with a specific medical history, lifestyle or genetic pool that could affect any of the organs or cells of the body at different levels with at present uncorrelated and uninvestigated impact on the spectra collected. Therefore, when studying human samples a source of variability related to each individual has to be tolerated while interpreting the data. However in most studies additional sources of variability, mostly instrumentation related, are added to the problem decreasing the specificity and reproducibility of the analysis. To discriminate between two populations often considered as normal and abnormal it is essential to identify markers specific to a pathological state but the importance of the control is often disregarded. In order to have a degree of relevancy the dataset representing the normal state of the sample is expected to exhibit a minimum level of variability. In other words, if the within group variability is greater than the between groups variability, the specificity of any discrimination reached has a limited degree of relevancy.

Unfortunately, results obtained previously have found a high degree of variability among cytology negative samples (normal samples). The present inventors have identified that contamination of the samples with blood residue is found to be present in a high percentage (close to 50%) of samples screened resulting in the variability seen. Accordingly, there is a need for a method of preparing biological samples which reduces the variability of the results, in particular when the samples are analysed by spectroscopy.

### Summary of the Invention

Accordingly, the invention provides a method of performing a spectroscopy process on a fixed biological sample wherein the biological sample is a cytological sample, the method comprising the steps of:
(i) treating the fixed biological material to cause oxidation of haemoglobin present in the fixed biological material, and
(ii) performing spectroscopy on the treated fixed biological sample.

Suitably, the spectroscopy process is Raman spectroscopy.

In the present invention Raman spectroscopy was used for the analysis of 50 normal cervical cell samples. The samples were prepared using the Thin prep method of sample preparation which is routinely used for screening of cervical cancer. The present invention has been described with reference to the use of the Thin prep method for preparing samples, however it will be appreciated by the person skilled in the art that the method described herein may also be used in the SurePath method of sample preparation and for treating samples from direct smears.

The inventors of the method according to the invention have identified that contamination of the samples with blood residue is found to be present in a high percentage (close to 50%) of samples screened. This contamination is not visible to the human eye or with a microscope but introduces significant variability in the spectra obtained. The variability generated within normal samples is such that it is too high to be able to use the data for a discrimination purpose between normal and abnormal samples.

Accordingly, to reduce the variability, a washing step is employed to clean the fixed samples. More particularly, the method according to the invention employs the use of a washing step with a solution comprising H₂O₂ (hydrogen peroxide).

Therefore, the step of treating the fixed biological material suitably comprises treating the fixed biological material with a solution comprising hydrogen peroxide. The fixed biological material suitably comprises cervical cells fixed on a microscope slide.

Preferably, the microscopic slide comprises a glass slide.

Whilst the spectra produced from samples is modified very slightly by the use of hydrogen peroxide, it has been found that the variability between "normal" samples is dramatically reduced. This provides for the realistic use of multivariate analysis of spectral results obtained using Raman spectroscopy in performing automatic detection between normal and abnormal cells.

Suitably the treatment employs a solution with a concentration of hydrogen peroxide in the range of 3% to 30%, preferably 15% to 30%, most preferably 30%.

The fixed biological material may be treated with the hydrogen peroxide for a period of between 2 and 5 minutes.

Preferably, the fixed biological material is treated with the hydrogen peroxide for a period of 5 minutes.

Hemoglobin is particularly sensitive to oxidation which irreversibly changes its structure and thus its spectral signature. Accordingly, the present invention comprises the immersion of individual samples on the slides into a solution of 30% H₂O₂ for approximately 5 minutes. The sample may be washed afterwards using alcohol.

Preferably, the method according to the invention further comprises the step of washing the slide with alcohol after treatment to remove hydrogen peroxide. The slide may be washed with any suitable alcohol such as methanol, ethanol or industrial methylated spirits, for example.

The method according to the present invention demonstrates the feasibility to record data from a large set of samples with good reproducibility. It has been shown that by washing the slides using H₂O₂ before recording the spectra, in addition to using adapted acquisition parameters (such as accumulation time for example) and optimising substrate removal methods (such as the removal of the spectral signal obtained from the glass slide, for example) it has been possible to greatly reduce the variability present in the data set. Adapted acquisition parameters and optimised substrate removal methods suitable for use in the method according to the invention are described herein.

For example, the method according to the invention suitably comprises the step of pre-exposing the fixed biological sample to a laser source for an accumulation time of 30 seconds during the spectroscopy process.

It will be appreciated by the person skilled in the art that the spectral features of glass are usually broad and can overlap with the region of interest in a spectrum obtained from a biological sample. The spectral signal generated from a glass substrate is therefore another source of variability when conducting spectroscopy on a sample. The invention further provides a method of correcting spectra in order to remove the contribution of the glass to the spectra.

Suitably, the method according to the invention may further comprise the steps of
(i) obtaining a spectrum and
(ii) compensating the spectrum to remove a spectral signal caused by the glass of the slide, the step of compensating comprising the step of reducing the intensity of said signal by a factor of 10 and repeating this step until a reference calculation is met.

The reference calculation suitably comprises comparing the ratio between two peaks in the spectrum.

Preferably the two peaks are at 1007cm⁻¹ and 1098cm⁻¹.
It has been demonstrated that the flexibility of the technique according to the invention will ease the integration in the medical environment as the protocols currently used routinely in cytology can be used for Raman spectroscopy.

### Brief Description of Drawings

The invention will now be described in detail by way of example and/or with reference to the accompanying drawings in which:

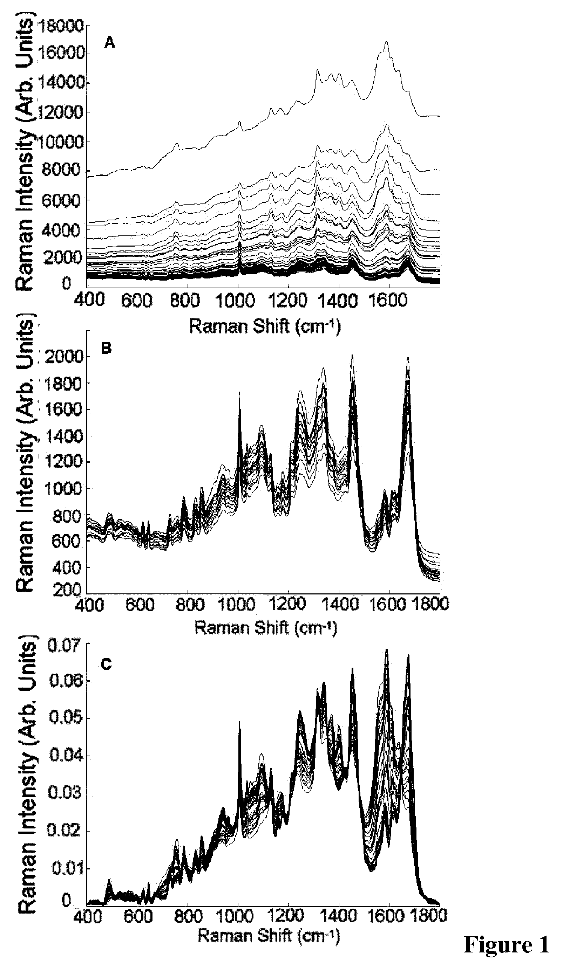

Figure 1 shows: A: Mean spectrum calculated for the raw spectra recorded from 50 normal samples. The black spectra exhibit regular cellular features (Pattern 1). The grey spectra represent the samples with a signature corresponding to abnormal features (Pattern 2); B: Mean spectra of the samples corresponding to the pattern 1. The light grey spectra have typical cellular features but can be discriminated due to a slight offset in their baseline; C: mean spectra of the 50 samples after pre-processing.

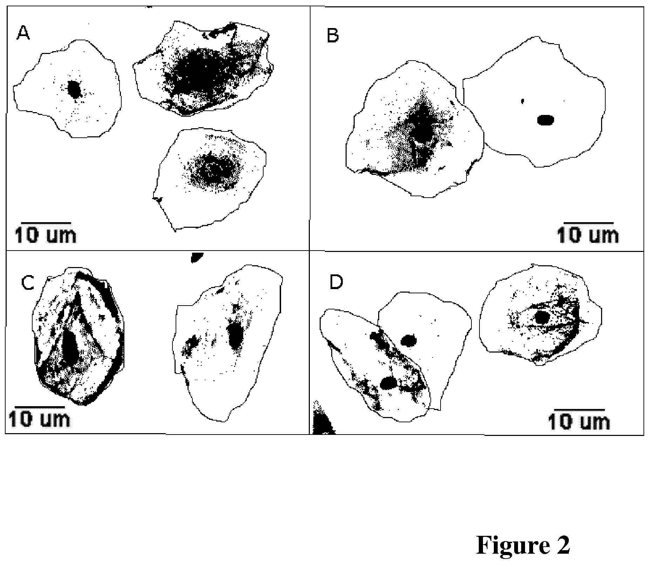

Figure 2 shows Thinprep samples as seen using an Olympus BX51 microscope equipped with a 40x objective. The cells have been stained with Haematoxylin Eosin. A: Cells from a Thinprep sample considered pattern 1 before treatment with H₂O₂; B: Cells from a Thinprep sample considered as pattern 1 after treatment with H₂O₂; C: cells from a Thinprep sample considered as pattern 2 before H₂O₂ treatment and D: cells from a Thinprep sample considered pattern 2 after H₂O₂ treatment.

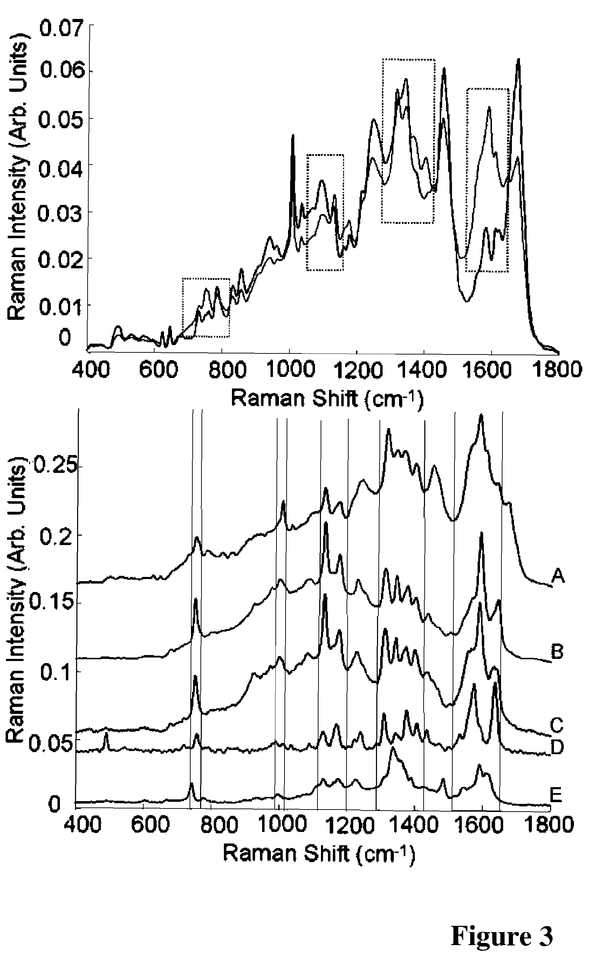

Figure 3 shows: Top: Comparison of the mean spectrum of the pattern 1 sample (black) and the pattern 2 samples (grey) Bottom: Comparison of the mean spectrum of the pattern 2 (A) with the spectra blood (B), haemoglobin (C), hemin (D) and proto-porphyrin (E).

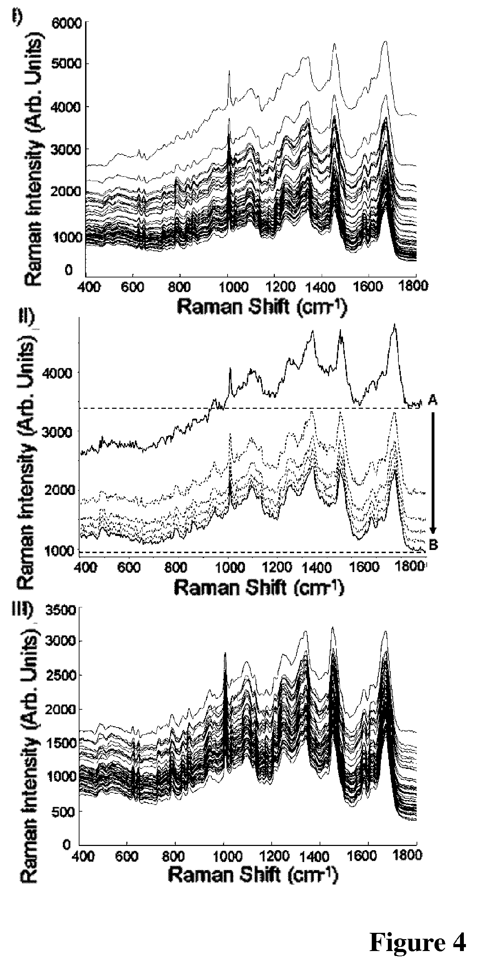

Figure 4 shows: I: Mean spectra from the 50 samples after treatment using H₂O₂. The black spectra already presented a pattern 1 before treatment. The grey spectra presented a pattern 2 before treatment. II: background decay for extended exposure to the laser. A: spectrum obtained after 10s accumulation. B: spectrum recorded after the 6^{th} accumulation of 10s. III: Mean spectra of the 50 samples after background removal using extended exposure to the laser.

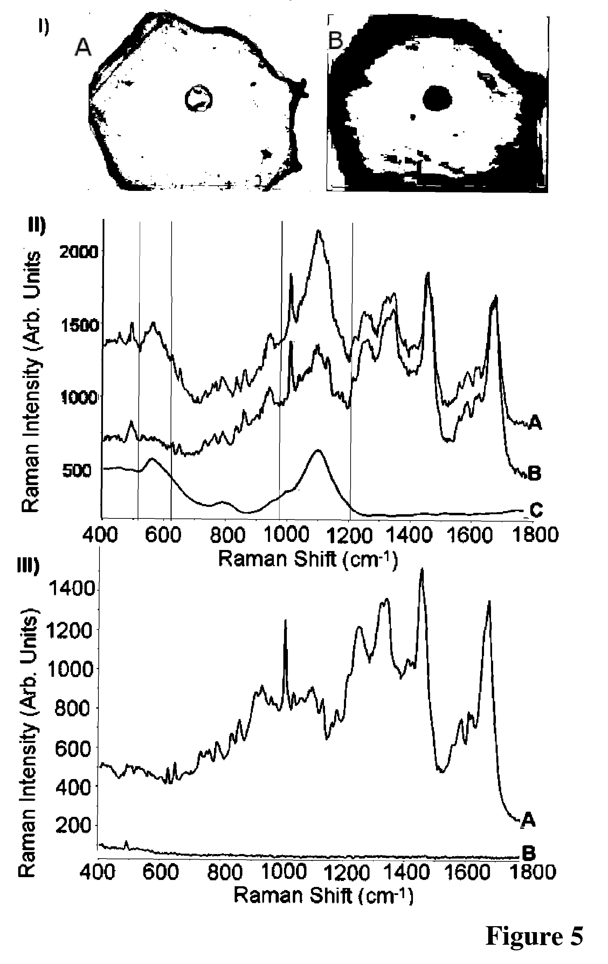

Figure 5: I: cells as seen on the Labram XploRA equipped with an 100x objective. A: focused on the cytoplasm, B: focused on the nucleus. II: Spectrum of nucleus from corresponding cell when focused on the cytoplasm (A) or the nucleus (B). C: spectrum of the glass substrate. III: A: Typical spectrum of a cervical cell deposed on a CaF₂ window, B: spectrum of the CaF₂ substrate.

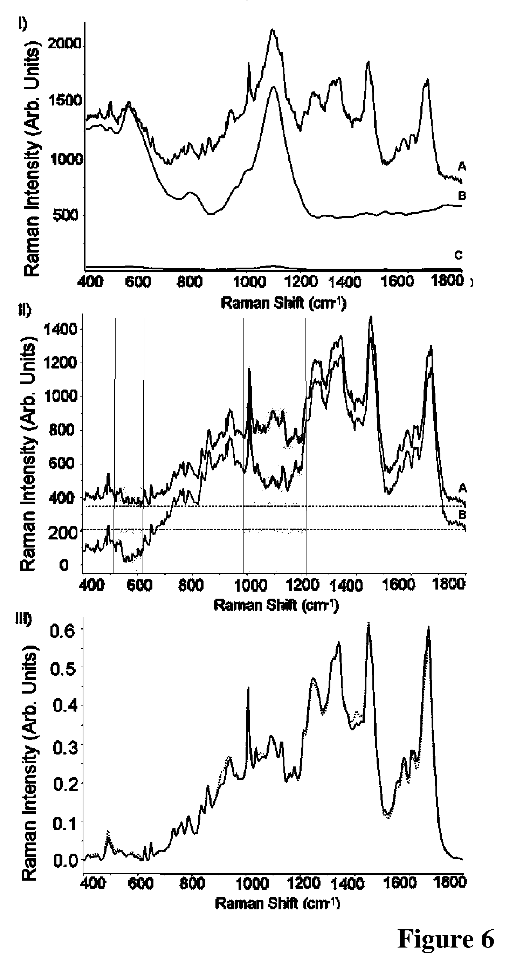

Figure 6: I) A: Example of cellular spectrum containing a strong contribution from the glass substrate. B: Typical glass signal expanded to match the intensity of the feature at 550-620 cm⁻¹. C: Same glass signal divided by a factor 10. II) A: Cellular spectrum recorded from a glass substrate after repeated subtraction of the glass background divided by a factor 10. B: Cellular spectrum recorded from a glass substrate after subtraction of glass background matching the intensity of the feature at 550-620 cm⁻¹. C: Cellular spectrum recorded from CaF₂ substrate (grey) compared to a cellular spectrum recorded from a glass substrate after successful subtraction of the glass contribution (black).

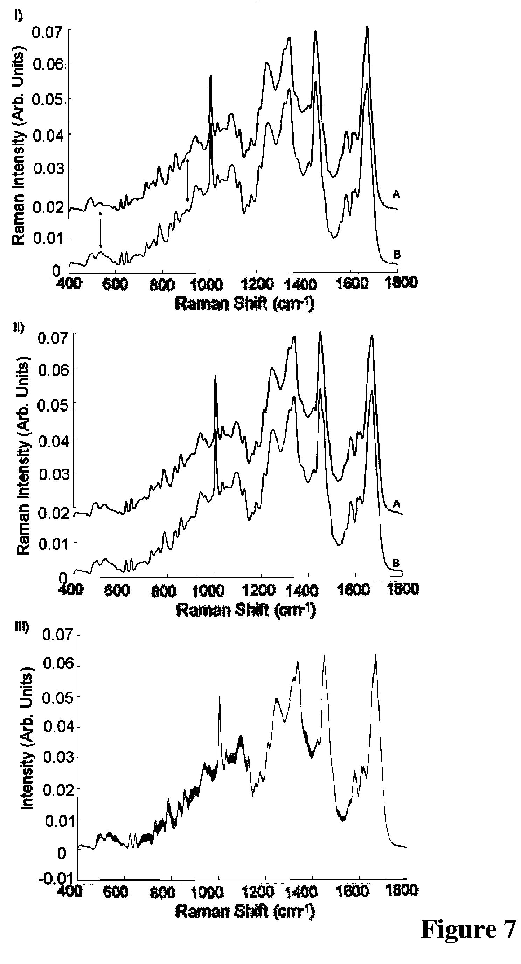

Figure 7: I) A: Mean spectrum of the pattern 1 sample before washing with H₂O₂. B: Mean spectrum of the pattern 1 samples after washing with H₂O₂. II) A: Mean spectrum of the pattern 1 sample after washing with H₂O₂. B: Mean spectrum of the pattern 2 samples after washing with H₂O₂. III) Mean spectrum of the whole data set after treatment with H₂O₂ with standard deviation calculated on for each spectra compared to the mean spectrum (shaded region).

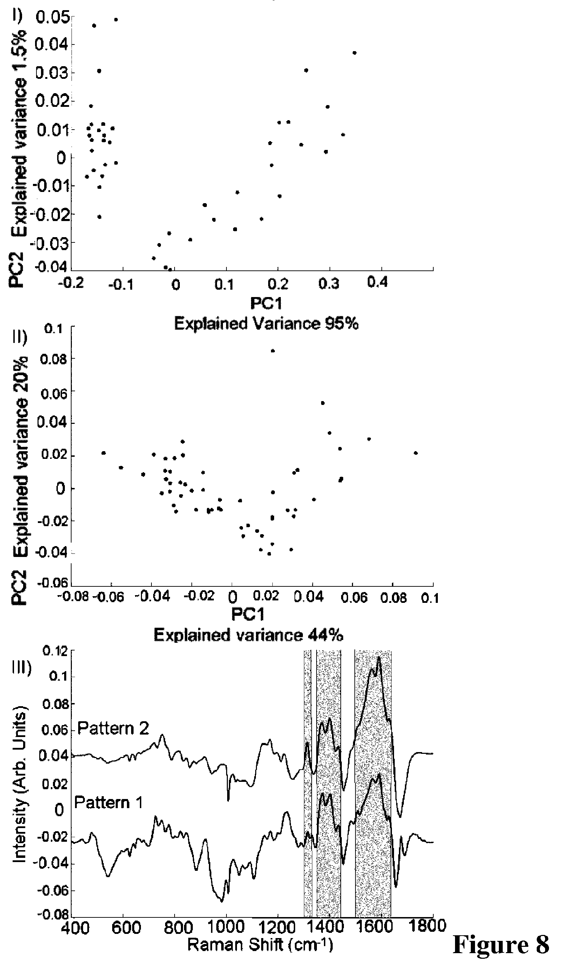

Figure 8: I) Scatter plot of the PCA analysis performed on the data recorded from Pattern 2 samples before (black) and after (grey) washing using H₂O₂. II) Scatter plot of the PCA analysis performed on the data recorded from Pattern 1 samples before (black) and after (grey) washing using H₂O₂. III) Loadings corresponding to the first PC for the pattern 1 and 2 data sets.

### Detailed Description of the Invention

The method according to the present invention is suitable for use with biological samples that have been fixed on a slide using liquid based cytology such as Thin prep and SurePath methods of sample preparation. It may also be used for direct smear samples.

The invention is described with reference to the following example in which the Thin prep method of preparing biological samples was used.

### Thin prep

The process begins with the patient's gynaecologic sample being collected by the clinician using either a broom-like device or a brush/plastic spatula combination. The device is then rinsed in the specimen vial. The vial suitably contains a solution for preserving the cells. One exemplary solution is the PreservCyt™ solution provided by Hologic of Massachusetts, USA. The sample is then homogenized by spinning. This spinning process may be performed by a specific device for the purpose, e.g. The T2000™ or T3000™ ThinPrep processor from Hologic. The spinning process is selected so as to create shear forces in the fluid that are strong enough to separate randomly joined material, break up blood, mucus and non-diagnostic debris while keeping true cell clusters intact. The cells may then be collected onto the membrane of the filter by applying a gentle vacuum across the filter membrane to aspirate fluid through the membrane. The resulting cells trapped in the filter may then be transferred to provide a monolayer of cells on a glass slide in a 20mm diameter circle. Once cell transfer is completed, the slide may be processed by immersion in a fixative bath containing 95% ethanol.

### Pure chemical preparation

Human hemoglobin, hemin and proto-porphyrin (lyophilized powder) were purchased from Sigma Aldrich (Ireland). Each of the chemical compounds was deposited on a CaF₂ window in the lyophilized form before recording.

An aliquot of blood comprising 200 microlitres of blood in 20ml Preservcyte solution was mixed in a ThinPrep vial containing no cervical cells, transferred onto a CaF₂ substrate and air dried before recording.

### Roman microspectroscopy

Raman studies were performed using a HORIBA Jobin Yvon XploRA™ system (Villeneuve d'Ascq, France), which incorporates an Olympus microscope BX41 equipped with a x100 objective (MPlanN, Olympus). A 532nm diode laser source was used throughout this work. In order to avoid any photo damage to the sample, the power of the laser was set at 50%, resulting in ~8 mW at the objective. The confocal hole was set at 100µm for all measurements, the specified setting for confocal operation. The system was pre-calibrated to the 520.7 cm⁻¹ spectral line of Silicon. The XploRA™ system is a confocal spectrometer that contains 4 interchangeable gratings (600, 1200, 1800 and 2400 lines/mm). In the present study the 1200 lines/mm grating was used, which gave a spectral dispersion of _{~}3 cm⁻¹ per pixel. Although a higher resolution grating such as 2400 gr/mm may give a spectral resolution of <1 cm⁻¹, the 1200 lines/mm grating was selected for this work to reduce the time of acquisition to 10s without decreasing the signal to noise ratio in the spectra collected. The backscattered light was measured using an air-cooled CCD detector (Andor, 1024 x 256 pixels). The spectrometer was controlled by Labspec V5.0 software. For each cell, 3 spectra were recorded from the nucleus, each of them corresponding to the average of 3 accumulations of 10s in the spectral range 400-1800 cm⁻¹. For each slide, 10 cells were selected randomly for recording. Also, for each slide, a spectrum of the substrate was recorded for correction of the data. Concerning the recording of the spectra from pure chemicals, the laser power was set to 1% in order to avoid photo-damage of the samples. The acquisition time was increased to provide a final signal to noise ratio close to the one seen on the cervical cells. Thus, the spectra presented are the results of 3 accumulations of 100s.

### Results and discussion

### 1. Roman spectroscopy of Thin Prep samples: sample variability

The average Raman spectra obtained from 50 patient samples with negative cytology are presented in the figure 1A. No pre-processing was applied to the data and no offset was added. During the screening of the different Thinprep samples, the presence of two different Raman signatures was clearly identified. This is unexpected, as the recordings have been performed specifically on the nucleus of the cells in order to reduce as much as possible the within group variability.

The application of Raman microspectroscopy for single cells *in vitro* has become very popular in the last decades, mainly due to the high resolution of the technique allowing access to sub-cellular information. Based on the numerous documents available in the literature, it is possible to determine what is a typical spectrum recorded from a single cell. Although Raman microspectroscopy can differentiate between different cell lines or cells in different phases of the cell cycle, the variations present in the spectra are usually minimal and can rarely be discerned by eye and the use of advanced multivariate analysis methods is required. Thus, in the present study, the black spectra in figure 1A can be assigned to a typical Raman signal recorded from cells (Bonnier *et al.* 2010, Draux *et al.* 2009) (pattern 1, n = 28) whereas the grey spectra are considered as atypical (pattern 2, n = 22). The main difference in the pattern 2 mean spectra is related to the presence of the broad background generating an offset of the baseline, more intense toward the high wavenumber region. Moreover, different features can be seen in the pattern 2 spectra which are not usually found in a typical cellular spectrum. Table 1 shows a comparison of the different peaks identified in both pattern 1 and pattern 2 spectra. A more detailed view of the pattern 1 samples can be found in figure 1B, which highlights the presence of a subgroup presenting similar features to pattern 1, but which exhibit a slightly higher background. For clarity, these spectra have been plotted in grey. This indicates that although the spectra are all recorded from normal cells, significant variability exists in the data sets recorded, depending on the sample quality and preparation. After data preprocessing, which includes a baseline correction followed by a vectorial normalization, the different mean spectra can be more easily compared (figure 1C). The different patterns can be clearly identified and the light grey spectra are now comparable to the pattern 1 spectra, which indicates the difference observed in the raw spectra was probably related to the morphology of the cells rather than the presence of a different Raman signature. The variability existing between the different samples can be greatly reduced after preprocessing and the spectra can be identified as being of two distinct patterns. Whereas the pattern 1 spectra are fairly consistent regarding the peak intensities, pattern 2 spectra show a significantly high degree of variability that can be clearly seen for the peaks located at 1642 cm⁻¹. Close inspection indicates the presence of an isosbestic-like point in the region of 1646 cm⁻¹ also visible at 1481 cm⁻¹. This indicates that a single spectral component is responsible for this specific Raman signature and it is present in different concentrations. However, when a pattern 2 signature is found on a sample, the entire cellular population will display the same profile, indicating that the effect is not related to the cell type recorded but is more likely cell suspension (patient) dependent.

**Table 1**

| **Pattern 1** | **Assignments** | **Pattern 2** |
|---|---|---|
| 497 | | 499 |
| 625 | C-C twisting mode of Phe (proteins) | 625 |
| 646 | C-C twisting mode of Tyr and Phe | 647 |
| 731 | C-N stretching in A and lipids | 732 |
| | **Pattern 2 feature** | **752** |
| 761 | Symmetric breathing of Trp (protein) | |
| 785 | U, T, C (ring breathing modes in the DNA/RNA) | 786 |
| 832 | PO2 stretching in DNA, Tyr | 831 |
| 857 | Ring breathing in Tyr and Pro (proteins) | 857 |
| 941 | C-C stretching mode of Pro and Val | |
| 961 | C-C and C-N stretch PO3 2- stretch (DNA) | |
| 1007 | C-C aromatic ring stretching in Phe | 1007 |
| | C-H bending mode in phenylalanine, C-N stretching in | |
| 1035 | proteins | 1036 |
| 1095 | Symmetric PO2 stretching of the DNA backbone; lipids | |
| 1129 | C-N stretching in proteins; C-O stretching in carbohydrates | 1132 |
| 1160 | C-C and C-N stretching of proteins | |
| 1177 | C-H in plane bending mode of Tyr and Phe; C, G | 1176 |
| 1212 | C-C6H5 stretching mode in Trp, Phe | |
| 1246 (1220-1280) | Amide III; A, C, T ring breathing modes of the DNA/RNA | 1244 |
| | **Pattern 2 feature** | 1316 |
| 1340 | G (DNA/RNA), CH deformation in proteins and carbohydrates | 1343 |
| | **Pattern 2 feature** | 1366 |
| | **Pattern 2 feature** | 1402 |
| 1424 | CH3 asymmetric stretch (lipids, aromatics) | |
| 1453 | CH (CH2) bending mode in proteins and lipids | 1453 |
| 1581 | A, G (DNA/RNA); C=C bending mode of Phe | |
| | **Pattern 2 feature** | 1588 |
| 1610 | C=C Phe, Tyr | 1608 |
| 1620 | C=C Tyr and Trp | |
| | **Pattern 2 feature** | 1642 |
| 1673 | Amide I | 1673 |

### 2. Understanding the origin of the pattern 2

Observation of the cells using an optical microscope does not give any indication of abnormal morphology of the cells. Figure 2A and 2C present a comparison of cells which exhibit respectively pattern 1 and pattern 2 Raman signatures, as seen using a Olympus BX51 microscope equipped with a 40x objective under white light. The cells have been stained with Haematoxylin/Eosin to facilitate the observation of the nucleus and membranes. The cells look perfectly normal in size and the nuclei have a perfectly circular shape and there are no perceptible morphological differences between samples classified as pattern 1 and pattern 2. As collection of the spectra was performed using a 100x objective giving a spot size of less than 1 µm at the focal point, the presence of small molecules at the surface of the cells not discernable by eye could easily contribute to the data collected, however. Thus, any contamination of the samples during the preparation of the cell suspension with the presence of bio-molecules could be seen as an abnormal Raman signature.
Figure 3 (top) presents the mean spectra obtained from the pattern 1 samples (black) and the pattern 2 samples (grey). The spectral windows exhibiting important variations in the Raman signatures have been highlighted by the black squares. A full list of the peaks and assignment can be found in Table 1. In order to understand the origin of the pattern 2 signature, the mean spectrum of a typical pattern 2 sample was compared to different spectra recorded from pure compounds (figure 3 (bottom)). The different spectral regions of interest have been highlighted in grey and the close similarity existing between the spectrum of the blood and the pattern 2 mean spectrum is clearly visible. The comparison with different molecules present in blood such as hemoglobin (figure 3C), hemin (figure 3D) or proto-porphyrin (figure 3E) validate this observation and the closest match is hemoglobin. Notably, hemoglobin is resonant at a wavelength of 532nm (Ivo *et al.* 2006, Ward *et al.* 2008), and thus the Raman signal is resonantly enhanced, such that even trace amounts can dominate the cellular signature. Thus, it may be concluded that, during the different steps of the sample preparation, the lysed blood present in the Thinprep suspension interacts with the cervical cells and can remain on the surface of the cells after drying. Although not visible by eye, the presence of hemoglobin can be detected by Raman spectroscopy and the spectral signature of this molecule is combined with the signal collected from the cells. Therefore, the example presented in figure 3A is mainly dominated by the hemoglobin signature and only a few specific features from the cells can be identified. Even the strong signature amide I band at ∼1650cm⁻¹ is almost completely obscured and appears only as a shoulder. The contamination of the samples with blood residue was found to be present in about 50 percent of the samples screened, and because almost half of the spectral range is affected, the specificity of the information contained in the spectra is greatly reduced. The variability generated within the normal samples (see figure 1C) is too high for the data to be used for the purpose of discrimination between normal and abnormal samples.

### 3. Dealing with the pattern 2: reversion to pattern 1

The origin of pattern 2 type spectra having been identified, the challenge is to remove the variability existing in the spectra to generate an acceptable control dataset. In order to solve the contamination of the samples with blood residues, three different approaches can be considered; (i) modify the protocol before the preparation of the samples while the cells are still in suspension, (ii) treat the slides and try to wash the cells after deposition and drying or (iii) manipulate the data and digitally remove the signal of the blood. Signal decomposition and correction with approaches such as Independent Component Analysis (ICA) coupled with Non-negatively Constrained Least Squares Analysis (NCLSA) has been documented and proven promising for the application to Raman data. The first step consists of identifying the independent components of pattern 2 by analyzing the co-variance existing in the data set. In the second step, the unwanted hemoglobin component is subtracted from the data set using NCLSA . This method has been successfully used to remove the contribution of wax in embedded skin sample for Raman and Infrared analysis (Tfayli *et al.* 2009, Ly *et al.* 2008). However, the samples exhibiting a pattern 2 signature have been also found to be more susceptible to photodegradation, causing some difficulties to collect the data from ThinPrep slides heavily contaminated with blood residues which could represent up to 5% of the samples screened. Thus, although the digital removal of the hemoglobin would be a valid approach, the aim of this invention was to develop an alternative approach allowing the collection of Raman data from all the different samples, regardless of the quantity of hemoglobin initially present on the cell surface. For this reason the present invention is focused on sample preparation and handling before the collection of the datasets.
Cell suspensions prepared for ThinPrep or smear samples are annotated according to a blood scale, whereby 0 indicates a clear solution and 3 indicates a bloody sample. In cytology laboratories, samples presenting a grade 2 or 3 on the blood scale are either rejected as being unsuitable or treated using Cytolyt or various solutions to wash the cells before preparing the samples. The Cytolyt solution is expensive and is usually used for bloody samples. It has been observed in this study that some samples with a grade 1 or 0 on the blood scale that would routinely be used for cytology present a pattern 2 signature. Therefore, for conformity with clinical procedures, all the samples would have to be washed with Cytolyt, which is unrealistic in terms of time and expense.

The only approach that can possibly be realistically applicable in a medical environment and for a large number of samples is the treatment of the slides after cell deposition and drying. To be present at this stage, the blood residues must have interacted with the cellular membrane, thus by destabilizing this interaction the residues may be washed away. Different solutions such as Cytolyt, eposti solution and acetic acid have been tested, but none delivered a reproducible result and features relating to the blood residue could still be seen in the spectra recorded (data not shown). Previous work published by Romeo *et al.* (2003) describes the feasibility to use red cell lysis buffer (RCLB) to reduce the presence of erythrocytes in cervical ThinPrep samples for IR spectroscopy. However, in our hands, this solution has been found to be inefficient after the cells have been deposited and dried on glass slides.
The action of H₂O₂ on the structure and conformation of hemoglobin is known (Vallelian *et al.* 2008, Zhou *et al.* 2007). Hemoglobin is particularly sensitive to oxidation, which irreversibly changes its structure. A solution of 30% H₂O₂ was therefore employed to treat the samples for 5 mins, after which the samples were washed in alcohol. The first concern was to preserve the cell morphology after such treatment. Figures 2B and 2D present cells stained using Haematoxylin/Eosin after treatment with H₂O₂. The cellular shape and size is not affected by the solution processing, probably due to the fact cells are fixed and dried on the glass slides. The use of staining is still achievable and the membrane and nucleus of the cells remain clearly identifiable. However, although the appearance of the cells appears unchanged, the molecular composition could be affected by such approach. Figure 4 presents the spectra collected from cells after H₂O₂ treatment. For consistency, all the slides have been submitted to the same protocols regardless of their initial spectral pattern. The grey spectra are those from cells originally presenting a pattern 2, exhibiting strong contributions of blood. These features are completely gone after washing and the only peaks visible are related to cellular components. Thus, the transformation from pattern 2 to pattern 1 is achievable using a short (5 minutes) exposure to H₂O₂ after drying of the samples. However, comparison with the black spectra, corresponding to the samples originally exhibiting a pattern 1, highlights the presence of a stronger background still remaining in the spectra recorded from samples originally presenting a pattern 2. Although the two datasets seem to be chemically comparable, they remain spectroscopically different. The problem of background in the data collected using Raman spectroscopy has been well documented in the literature (Mazet *et al.* 2005, Zhao *et al.* 2007, Zhang *et al.* 2010). Most of the papers describe the phenomenon as being related to fluorescence. Recent work based on the analysis of human skin has also described the presence of background in the spectra recorded but suggested that the morphology of the samples is a determining factor (Bonnier *et al.* 2011). Moreover, it has been demonstrated that prolonged exposure to the laser source can gradually decrease the background seen in the spectra recorded (Bonnier *et al.* 2012). Similar effects can be seen in the present study and figure 4II presents an example of the background decay over time. A set of 6 spectra has been recorded on the same spot, without any movement of the sample, using an accumulation time of 10s. Spectrum A represents the signal originally recorded. The background creates a distortion of the baseline that can be seen with the black dotted line. The last spectrum (B) corresponds to the 6^{th} accumulation of 10s acquired from this spot of the sample. The background is completely gone and the spectrum displays a profile similar to those recorded for cells grown *in vitro* (Notingher *et al.* 2006, Draux *et al.* 2009, Bonnier *et al.* 2010). The grey spectra represent the intermediate states for each accumulation of 10s. After 30s exposure to the laser, the background becomes relatively constant and the decrease is minimal. Therefore pre-exposure to the laser of 30s has been employed for any further measurement carried out on the ThinPrep samples.
Figure 4III presents the mean spectra obtained from the 50 samples using the H₂O₂ treatment and the 30s (seconds) pre-exposure to the laser. The variability existing between the data sets corresponding to the samples originally displaying pattern 1 (black) and pattern 2 (grey) has been greatly reduced. Although the intensity is slightly different, the background remains similar. Thus, all the spectra have a profile corresponding to those commonly observed on cells grown *in vitro.*

### 4. Impact of the system confocality on the data collected

In addition to the chemical contamination of the samples, other parameters can induce variability in the data sets recorded. For instance, as the cellular material tends to be relatively thin, the choice of substrate is critical. The spectral features of glass are usually broad and can overlap significantly with the region of interest of the cellular spectra and can therefore be another source of variability (Bonnier *et al.* 2010, 2011). Quartz or CaF₂ are commonly used as an alternative, as, although these substrates can contribute to the final spectra, they do not contain any specific sharp peaks which interfere with the cellular spectrum and their contribution can be at least partially removed by a subtraction (Draux *et al.* 2009). However, the cost of Quartz or CaF₂ slides is simply unrealistic for screening large numbers of samples. Moreover, as the present invention demonstrates the potential to apply Raman micro spectroscopy for the screening of cervical ThinPrep samples, it is therefore important to develop a technique compatible with the protocols used in the medical field. Thus, glass slides have been used as substrate throughout this work, and all the samples are prepared on ThinPrep glass slides for cytology. Notably, although it may be argued that the contributions of residual hemoglobin would be greatly reduced by moving to longer, nonresonant wavelengths such as 785nm, commonly employed for Raman analysis of biological samples, it was found that the glass slides exhibited a substantial background at this wavelength. Furthermore, using the 532 nm wavelength as a source, the confocality of the Raman can be greatly improved and the contribution from the substrate significantly decreased. The Raman microscope is equipped with a 100x objective and the different structures of the cells are easily discernable. Figure 5IA presents an image of a cell as seen on the Raman system. The nucleus and membrane are clearly visible; however the laser can be focused either on the cytoplasm (figure 5IA) or the nucleus (figure 5IB). This results in significant changes in the contribution of the glass to the spectra collected, as seen in figure 5II. The spectrum of the glass is represented by spectrum C and the two main features are seen between 550-620 cm⁻¹ and 1000-1200 cm⁻¹. These broad features can also be seen in the spectra collected from the cell, but the intensity is greatly reduced when the focus is on the nucleus (spectrum B) rather than the cytoplasm (spectrum A).

For comparison, samples have been prepared on CaF₂ using the Thinprep protocol. The weak contribution of the CaF₂ in the spectra collected (figure 5III B) allows visualisation of the cellular spectrum without the contribution of the glass (figure 5III A)

### 5. Substrate removal from the data set

Figure 6IA presents an example of a spectrum recorded from the nucleus of a cervical cell with a strong contribution from the glass substrate as presented in figure 5IIIA. This corresponds to a focus on the cytoplasm rather than the nucleus. Although during recording of the data the operator was careful to select an appropriate focus to have an optimal signal to background ratio with minimum contribution from the substrate, this example was selected to demonstrate the feasibility to remove the signal of the glass from the data recorded. The main difficulty is to estimate the contribution from the glass in the spectrum recorded. The laser being focused on the cell, it will be influenced by the density of the sample and how far the laser is from the surface of the glass. Because each cell has a different morphology and a different thickness, it is considered that every spectrum will have a different contribution from the glass. It is unlikely to have a perfect mix corresponding to 50% cellular features, 50% glass features. Based on the focus used to record the cellular spectrum, it is possible to move the stage beside the cell and record a spectrum of the glass with the same Z position; however the absence of biological material in the path of the laser makes the intensity of the signal collected from the substrate not suitable for correction. Therefore, the best approach remains to collect a spectrum from the glass, with the laser focused on the glass and try to match the intensity according to the intensity of the glass features. As shown in figure 6IB, the intensity of the glass spectrum has been matched with the intensity of the glass feature visible between 550-620 cm⁻¹. Due to the different band ratios, the feature around 550 cm⁻¹ seemed to be the most suitable in order to not have a greater intensity in the spectrum of the glass than the cellular spectrum which will result in negative values. However, after subtraction, the result demonstrates the lack of accuracy of this method. The spectrum seems over corrected compared to a spectrum recorded from the CaF₂ substrate (Figure 5IIIA). The baseline appears distorted, as shown by the dotted line (figure 6IIA). Similar attempts can be made using the band around 1100 cm⁻¹, but the resulting spectrum appears even more over corrected (data not shown).
Rather than trying to match the intensity of the glass signal with the intensity of the cellular spectrum, the second approach consists of reducing its intensity by a factor of 10. Thus, the intensity of the glass signal becomes small compared to the spectrum of the nucleus (figure 6IC). Because it is difficult to estimate how much the glass contributes in the spectra collected from the cells, it has been considered that repeated subtraction of the signal of the glass divided by 10 will allow to fine tune the correction and after a number of subtractions, x, the glass will be completely removed without any visual effect of over-correction. However, in order to estimate when the glass has been completely removed a control was needed. For this reason, three fresh samples were prepared on both glass and CaF₂ windows and recorded in similar conditions to the ThinPrep samples. To evaluate the presence of glass in the spectrum the band ratio 1007/1098 cm⁻¹ has been used. The ratio has been calculated for the entire data set recorded from the CaF₂ substrates and the average value has been used as the target for the glass correction. In order to compensate for the glass signal and to ensure the glass signal is completely removed, a reference calculation may be used. For example, using an algorithm in Matlab, the signal of the glass divided by 10 has been successively subtracted from the data until the ratio of the peaks at 1007/1098 cm⁻¹ matches the ratio obtained on the CaF₂ (Figure 6IIA). The resulting mean spectrum after glass correction and pre-processing (baseline correction and normalization) is displayed in figure 6III in black, compared to the mean spectrum obtained from the CaF₂ substrate. After correction, the two spectra have perfectly comparable profiles and no contribution from the glass can be seen, which indicates that the removal has been successful. This method has been used for the correction of the substrate contribution in the following steps of the study.

### 6. Evaluation of the sample variability after pre-processing

H₂O₂ is a strong reactive species and has an oxidizing effect on proteins. Thus, it has been necessary to first make sure the cells were not affected by the treatment. Although the cell morphology is intact after treatment and no visual alteration of the composition can be seen (figure 2) the molecular organization can be slightly different and have an impact on the spectra recorded. As an internal control, the samples presenting a pattern 1 signature before the H₂O₂ washing were used. Figure 7 I) A presents the mean spectrum of the pattern 1 samples before treatment compared to the mean spectrum of the pattern 1 samples after treatment (figure 7 I) B). The two spectra display strong similarities and only two small bands located at 536 cm⁻¹ and 904 cm⁻¹ have their intensities changed. Apart from those peaks, no noticeable variability can be observed in the spectra. Indeed, the overlap between the two spectra is not perfect, but as the cells are picked randomly, some variability has to be expected. The study of the data sets before and after washing with H₂O₂ using PCA gives additional understanding of the origin of any spectral variability. Figure 8 I) displays the PCA scatter plot based on the pattern 2 samples before and after washing, highlighting a clear discrimination between the 2 datasets which according to the loading of PC1, plotted in figure 8 III) (marked pattern 2), is due to the removal of the hemoglobin from the samples. However, after performing a PCA on the datasets described as pattern 1, the scatter plot also displays a strong discrimination between the spectra recorded before and after washing using H₂O₂. For comparison, the loading corresponding to PC1 has been plotted in figure 8 III) (marked pattern 1). The similarities existing between the loadings calculated from the samples classified as pattern 1 and 2 are notable. This demonstrates that the patterns have been arbitrarily associated to the samples for convenience through the study but although some samples didn't exhibit strong features from the hemoglobin and were considered as pattern 1 the spectra were influenced by underlying contributions which mixed with the complex signal from the cellular features was undetectable by eye. However, this can be detected using a statistical analysis approach such as PCA. Thus, the variation seen in the pattern 1 mean spectra plotted in figure 7 I) A and 7 I) B can be more likely assigned to the washing of small hemoglobin residues present on the cell surface.
More importantly, the variability existing between the samples originally exhibiting a pattern 1 signature and a pattern 2 signature has to be investigated after treatment with H₂O₂. Figure 7 II) presents the mean spectrum of the samples with a pattern 1 signature before washing (black), compared to the mean spectrum of the samples with a pattern 2 signature after washing (grey). The spectra have been baseline corrected and normalized for comparison. The spectral features are perfectly comparable and no alteration of the molecular composition of the cells can be seen. Figure 7 III) is an attempt to represent the overall quality of the data set after treatment with H₂O₂. The mean spectrum after baseline correction and vector normalisation has been plotted in black, surrounded by the standard deviation represented by the grey shade. As a comparison, the initial data displayed in figure 1C had a greater degree of variability. Thus, starting with 2 distinct signatures, obviously different, the adaptation of the sample preparation steps and the glass subtraction resulted in a significantly reduced within group variability which will give the possibility to perform accurate and more specific analysis of the ThinPrep samples using Raman spectroscopy. However, the cellular variability remains one of the most limiting factors. Thus, when collecting spectra from different cells, differences in the cell morphology, size, but also metabolism or phenotype at the moment of the sample preparation can have a significant impact on the features present in the data. The use of a 100x objective gives access to subcellular analysis of single cells but using wavelength such as 532 nm, the spot being reduced to less than a micron, the sub-nuclear analysis of the cells is also possible (Bonnier *et al.* 2010). While the identification of the nucleolus can be achieved when working on cancerous cell lines grown on substrates (Miljkovic *et al.* 2010, Dorney *et al.* 2012), in the case of cervical cells prepared as ThinPrep slides, the nucleus doesn't present any evidence of sub-nuclear organization, although not being visibly discernible does not necessarily mean the molecular composition of the nucleus is homogeneous. Therefore, although 3 spectra have been recorded for each cell in order to reduce the variability due to random selection of the collection spots; most of the nucleus is not being recorded due to the high magnification offered by the use of a 100x objective. Thus, the remaining standard deviation observed in the data sets is likely to be cell to cell variability due the presence of sub-nuclear structures inducing subtle modifications of the spectral features present in the data sets. This remaining variability can only be removed by adapting the approach used for the spectral data collection and improvement of the nuclear area covered during the spectra collection.

The variability present in patient samples can always be an issue for the potential discrimination between normal samples and pathological or abnormal samples. Although it is often admitted that more specific analytical methods are needed to extract the relevant information, it is often forgotten that the sample preparation can have a crucial place in the success of such studies. Concerning the study of Thinprep samples using Raman spectroscopy, the presence of blood can be the limiting parameter as its spectral signatures overlap the cellular features therefore making the use of the raw data impossible for diagnostic purposes. However, the present invention clearly demonstrates that rather than try to develop advanced algorithms of multivariate analytical methods to correct the data, treatment of the samples can wash off the blood residue present on the cell membrane and, together with adapted background reduction and substrate removal methods, the collection of highly reproducible data can be achieved.

The words comprises/comprising when used herein are to specify the presence of stated features, integers, steps or components but do not preclude the presence or addition of one or more other features, integers, steps, components or groups thereof.

### References

Bonnier F, Ali SM, Knief P, Lambkin H, Flynn K, McDonaghc V, Healy C, Lee TC, Lyng FM, Byrne HJ, Vibrational Spectroscopy, 2012, 61, 124-132.
Bonnier F, Knief P, Lim B, Meade AD, Dorney J, Bhattacharya K, Lyng FM, Byrne HJ, Analyst, 2010, 135, 3169-3177.
Bonnier F, P. Knief, Meade AD, Dorney J, Bhattacharya K, Lyng FM, Byrne HJ. 2011. Collagen matrices as an improved model for in vitro study of live cells using Raman microspectroscopy in Clinical and Biomedical Spectroscopy and Imaging II in Ramanujam N, Popp J, eds. SPIE-OSA Biomedical Optics Munich (Germany).
Bonnier F, Meade AD, Merzha S, Knief P, Bhattacharya K, Lyng FM, Byrne HJ, Analyst, 2010, 135, 1697-1703.
Bonnier F, Mehmood A, Knief P, Meade AD, Hornebeck W, Lambkin H, Flynn K, McDonagh V, Healy C, Lee TC, Lyng FM, Byrne HJ, Journal of Raman Spectroscopy, 2011, 42, 888-896.
Dorney J, Bonnier F, Garcia A, Casey A, Chambers G, Byrne HJ, Analyst, 2012, 137, 1111-1119.
Draux F, Jeannesson P, Beljebbar A, Tfayli A, Fourre N, Manfait M, Sule-Suso J, Sockalingum GD, Analyst, 2009, 134, 542-548.
Ivo P. Torres Filho, James Terner, Roland N. Pittman, Elizabeth Proffitt, Ward KR, J Appl Physiol, 2008, 104, 1809-1817.
Ly E, Piot O, Wolthuis R, Durlach A, Bernard P, Manfait M, Analyst, 2008, 133, 197-205.
Lyng FM, Faolain EO, Conroy J, Meade AD, Knief P, Duffy B, Hunter MB, Byrne JM, Kelehan P, Byrne HJ, Exp Mol Pathol, 2007, 82, 121-129.
Mazet V, Carteret C, Brie D, Idier J, Humbert B, Chemometrics and Intelligent Laboratory Systems, 2005, 76, 121.
Miljkovic M, Chernenko T, Romeo MJ, Bird B, Matthaus C, Diem M, Analyst, 2010, 135,2002-2013.
Notingher I, Hench LL, Expert Rev Med Devices, 2006, 3, 215-234.
Romeo MJ, Wood BR, Quinn MA, McNaughton D, Biopolymers (Biospectroscopy), 2003, 72, 69-76.
Tfayli A, Gobinet C, Vrabie V, Huez R, Manfait M, Piot O, Appl Spectrosc, 2009, 63, 564-570.
Vallelian F, Pimenova T, Pereira CP, Abraham B, Mikolajczyk MG, Schoedon G, Zenobi R, Alayash AI, Buehler PW, Schaer DJ, Free Radic Biol Med, 2008, 45, 1150-1158. Ward KR, Torres Filho I, Barbee RW, Torres L, Tiba MH, Reynolds PS, Pittman RN, Ivatury RR, Terner J, Crit Care Med, 2006, 34, 792-799.
Wong PT, Wong RK, Caputo TA, Godwin TA, Rigas B, Proc Natl Acad Sci USA, 1991, 88, 10988-10992
Zhao J, Lui H, McLean DI, Zeng H, Appl Spectrosc, 2007, 61, 1225-1232.
Zhang, Z.-M., Chen, S., Liang, Y.-Z., Liu, Z.-X., Zhang, Q.-M., Ding, L.-X., Ye, F. and Zhou, H. Journal of Raman spectroscopy, 2010, 41, 659-669.
Zhou SL, Wang JH, Huang WH, Lu X, Cheng JK, J Chromatogr B Analyt Technol Biomed Life Sci, 2007, 850, 343-347.

## Claims

1. A method of performing a spectroscopy process on a fixed biological sample wherein the biological sample is a cytological sample,
the method comprising the steps of:
(i) treating the fixed biological material to cause oxidation of haemoglobin present in the fixed biological material, and
(ii) performing spectroscopy on the treated fixed biological sample.

2. A method according to claim 1, wherein the spectroscopy process is Raman spectroscopy.

3. A method according to claim 1 or 2, wherein the step of treating the fixed biological material comprises treating the fixed biological material with a solution comprising hydrogen peroxide.

4. A method according to claim 3, further comprising the step of washing the slide with alcohol after treatment to remove hydrogen peroxide.

5. A method according to any one of claims 1 to 4, wherein the treatment employs a solution with a concentration of hydrogen peroxide in the range of 3% to 30%, preferably 15% to 30%, most preferably 30%.

6. A method according to claim 5, wherein the fixed biological material is treated with the hydrogen peroxide for a period of between 2 and 5 minutes.

7. A method according to claim 6 wherein the fixed biological material is treated by the hydrogen peroxide for a period of 5 minutes.

8. A method according to any one of claims 1 to 7, wherein the biological material comprises cervical cells fixed on a microscope slide.

9. A method according to claim 8 wherein the microscopic slide comprises a glass slide.

10. A method according to any preceding claim, further comprising the step of pre-exposing the fixed biological sample to a laser source for an accumulation time of 30 seconds during the spectroscopy process.

11. A method according to claim 10 further comprising the steps of
(i) obtaining a spectrum and
(ii) compensating the spectrum to remove a spectral signal caused by the glass of the slide, the step of compensating comprising the step of reducing the intensity of said signal by a factor of 10 and repeating this step until a reference calculation is met.

12. A method according to claim 11 wherein the reference calculation comprises comparing the ratio between two peaks in the spectrum.

13. A method according to claim 12 wherein the two peaks are at 1007cm⁻¹ and 1098cm⁻¹

## Patentansprüche

1. Verfahren zum Ausführen eines Spektroskopie-Prozesses auf eine fixierte biologische Probe, wobei die biologische Probe eine zytologische Probe ist, wobei das Verfahren folgende Schritte umfasst:
(i) Behandlung des fixierten biologischen Materials zur Veranlassung einer Oxidation von Hämoglobin, das in dem fixierten biologischen Material vorliegt, und
(ii) Ausführen einer Spektroskopie bezüglich der behandelten fixierten biologischen Probe.

2. Verfahren gemäß Anspruch 1, wobei der Spektroskopie-Prozess eine Raman-Spektroskopie ist.

3. Verfahren gemäß Anspruch 1 oder 2, wobei der Schritt einer Behandlung des fixierten biologischen Materials ein Behandeln des fixierten biologischen Materials mit einer Lösung umfasst, die Wasserstoffperoxid aufweist.

4. Verfahren gemäß Anspruch 3, ferner umfassend den Schritt eines Waschens des Objektträgers mit Alkohol zur Entfernung von Wasserstoffperoxid nachfolgend der Behandlung.

5. Verfahren gemäß einem der Ansprüche 1 - 4, wobei bei der Behandlung eine Lösung mit einer Konzentration von Wasserstoffperoxid in dem Bereich von 3% bis 30%, vorzugsweise 15% bis 30%, am bevorzugtesten 30% eingesetzt wird.

6. Verfahren gemäß Anspruch 5, wobei das fixierte biologische Material für eine Zeitdauer von zwischen 2 und 5 Minuten mit dem Wasserstoffperoxid behandelt wird.

7. Verfahren gemäß Anspruch 6, wobei das fixierte biologische Material für eine Zeitdauer von 5 Minuten durch das Wasserstoffperoxid behandelt wird.

8. Verfahren gemäß einem der Ansprüche 1 - 7, wobei das biologische Material Gebärmutterhals-Zellen aufweist, die auf einem Mikroskop-Objektträger fixiert sind.

9. Verfahren gemäß Anspruch 8, wobei der Mikroskop-Objektträger eine Glasplatte umfasst.

10. Verfahren gemäß einem der vorangehenden Ansprüche, ferner umfassend den Schritt einer Freisetzung der fixierten biologischen Probe an eine Laserquelle für eine Akkommodationszeit von 30 Sekunden während des Spektroskopie-Prozesses.

11. Verfahren gemäß Anspruch 10, ferner umfassend die Schritte eines
(i) Erhaltens eines Spektrums, und
(ii) Kompensierens des Spektrums zum Beseitigen eines Spektralsignals, das durch das Glas des Objektträgers verursacht ist, wobei der Schritt des Kompensierens den Schritt eines Reduzierens der Intensität des besagten Signals durch einen Faktor 10 umfasst, und ein Wiederholen dieses Schrittes bis eine Referenz-Berechnung übereinstimmend vorliegt.

12. Verfahren gemäß Anspruch 11, wobei die Referenzberechnung ein Vergleichen des Verhältnisses zwischen zwei Peaks in dem Spektrum umfasst.

13. Verfahren gemäß Anspruch 12, wobei die zwei Peaks bei 1007 cm⁻¹ und 1098 cm⁻¹ liegen.

## Revendications

1. Méthode pour la réalisation d'un procédé de spectroscopie sur un échantillon biologique fixé, dans laquelle l'échantillon biologique est un échantillon cytologique,
ladite méthode comprenant les étapes consistant à :
(i) traiter la matière biologique fixée afin de provoquer l'oxydation de l'hémoglobine présente dans la matière biologique fixée, et
(ii) réaliser une spectroscopie sur l'échantillon biologique fixé traité.

2. Méthode selon la revendication 1, dans laquelle le procédé de spectroscopie est la spectroscopie Raman.

3. Méthode selon la revendication 1 ou 2, dans laquelle l'étape de traitement de la matière biologique fixée comprend le traitement de la matière biologique fixée avec une solution comprenant du peroxyde d'hydrogène.

4. Méthode selon la revendication 3, comprenant en outre l'étape de lavage de la lame avec de l'alcool après traitement pour retirer le peroxyde d'hydrogène.

5. Méthode selon l'une quelconque des revendications 1 à 4, dans laquelle le traitement utilise une solution ayant une concentration en peroxyde d'hydrogène dans la plage de 3 % à 30 %, de préférence de 15 % à 30 %, le plus préférentiellement de 30 %.

6. Méthode selon la revendication 5, dans laquelle la matière biologique fixée est traitée avec le peroxyde d'hydrogène pendant une durée située entre 2 et 5 minutes.

7. Méthode selon la revendication 6, dans laquelle la matière biologique fixée est traitée par le peroxyde d'hydrogène pendant une durée de 5 minutes.

8. Méthode selon l'une quelconque des revendications 1 à 7, dans laquelle la matière biologique comprend des cellules cervicales fixées sur une lame de microscope.

9. Méthode selon la revendication 8, dans laquelle la lame de microscope comprend une lame de verre.

10. Méthode selon l'une quelconque des revendications précédentes, comprenant en outre l'étape de pré-exposition de l'échantillon biologique fixé à une source de laser pour un temps d'accumulation de 30 secondes au cours du procédé de spectroscopie.

11. Méthode selon la revendication 10, comprenant en outre les étapes de
(i) obtention d'un spectre et
(ii) compensation du spectre pour éliminer un signal spectral provoqué par le verre de la lame, l'étape de compensation comprenant l'étape de réduction dudit signal d'un facteur de 10 et de répétition de cette étape jusqu'à ce qu'une condition donnée par un calcul de référence soit satisfaite.

12. Méthode selon la revendication 11, dans laquelle le calcul de référence comprend la comparaison du rapport entre deux pics dans le spectre.

13. Méthode selon la revendication 12, dans laquelle les deux pics sont à 1 007 cm⁻¹ et 1 098 cm⁻¹
